# EUROPEAN PATENT SPECIFICATION

(11) **EP 2 326 333 B1**
(45) Date of publication and mention of the grant of the patent: **31.05.2017**
(21) Application number: 09806548.5
(22) Date of filing: 08.07.2009
(51) Int. Cl.: A61K 31/7088, A61K 38/00, A61P 9/10, A61P 25/00, A61P 25/14, A61P 25/28, C07K 14/47, C07K 14/705, C07K 19/00, C12N 15/09, C12N 15/113

(54) **AGENT FOR PROMOTING NEURONAL DIFFERENTIATION AND METHOD THEREFOR**
MITTEL ZUR FÖRDERUNG VON NEURONALER DIFFERENZIERUNG UND VERFAHREN DAFÜR
AGENT FAVORISANT LA DIFFÉRENTIATION NEURONALE ET PROCÉDÉ ASSOCIÉ

(30) Priority: 13.08.2008 US 88521 P
(43) Date of publication of application: 01.06.2011
(73) Proprietor: Keio University, Tokyo 108-8345 (JP)
(72) Inventor: OKANO, Hideyuki, Tokyo 160-8582 (JP); SHIMAZAKI, Takuya, Tokyo 160-8582 (JP); NAKA, Hayato, Tokyo 160-8582 (JP)
(74) Representative: Gill Jennings & Every LLP
(86) International application number: PCT/JP2009/003195
(87) International publication number: WO 2010/018652

(56) References cited:
- WO-A1-01/57191
- WO-A1-99/06034
- WO-A2-03/046141
- WO-A2-2005/017112
- KANAI M I ET AL.: 'seven-up controls switching of transcription factors that specify temporal identities of Drosophila neuroblasts.' DEVELOPMENTAL CELL vol. 8, 2005, pages 203 - 213, XP008143838
- POULSEN C B ET AL.: 'Brain response to traumatic brain injury in wild-type and interleukin-6 knockout mice: a microarray analysis.' J. NEUROCHEM. vol. 92, 2005, pages 417 - 432, XP008143920
- ZHUANG Y ET AL.: 'Overexpression of COUP-TF1 in murine embryonic stem cells reduces retinoic acid-associated growth arrest and increases extraembryonic endoderm gene expression.' DIFFERENTIATION vol. 76, 03 January 2008, pages 760 - 771, XP026772229
- FAEDO A ET AL.: 'COUP-TFI coordinates cortical patterning, neurogenesis, and laminar fate and modulates MAPK/ERK, AKT, and beta-catenin signaling.' CEREBRAL CORTEX vol. 18, 28 December 2007, pages 2117 - 2131, XP008143925
- YAMAGUCHI H ET AL.: 'The nuclear orphan receptor COUP-TFI is important for differentiation of oligodendrocytes.' DEV. BIOL. vol. 266, 2004, pages 238 - 251, XP008144819
- NAKA H ET AL.: 'Requirement for COUP-TFI and II in the temporal specification of neural stem cells in CNS development.' NAT. NEUROSCI. vol. 11, 24 August 2008, pages 1014 - 1023, XP008144820
- OKADA Y ET AL.: 'Spatiotemporal recapitulation of central nervous system development by murine embryonic stem cell-derived neural stem/progenitor cells.' STEM CELLS vol. 26, 28 August 2008, pages 3086 - 3098, XP008143940

## Description

### [Technical field]

The present invention relates to agents for use in a method of treatment for promoting neuronal differentiation of neural stem/progenitor cells in vivo.

### [Background Art]

In order to utilize stem cells having multipotency in the field of regenerative medicine, it is necessary to differentiate the stem cells into a certain cell type. Neural stem/progenitor cells (NCPCs) can differentiate from embryonic stem cells (ES cells) in vitro (Japanese Patent Application Laid-open Publication No.2002-291469), and also can be isolated from an embryonic brain (Reynolds, B.A. and Weiss, S. Science vol.255, pp.1707-1710 (1992)). The NCPCs are capable of differentiating into both neurons and glial cells, and methods for inducing the differentiation into either neurons or glia have been developed.

For example, by administering an IL-6 family cytokine (Koblar, S.A., et al. Proc.Natl.Acad.Sci.U.S.A. vol.95, pp.3178-3181 (1998)) or BMP2/4 (Gross, R.E., et al. Neuron vol.17, pp.595-606 (1996)) to NCPCs, they can be induced to differentiate into glial cells.

However, mechanisms of the differentiation into neurons, especially of sequential differentiation into different types of neurons have been scarcely elucidated, and thus the techniques for differentiating specifically into neurons have not been as much developed as those for glial cells.

### [Summary of Invention]

### [Technical Problem]

The present invention is intended to provide agents for promoting neuronal differentiation of NSPCs, and methods therefor.

### [Solution to Problem]

In one embodiment of the present invention, an agent for use in a method of treatment that promotes neuronal differentiation of an NSPC includes an inhibitor of function of a COUP-TFI protein and/or a COUP-TFII protein. The inhibitor may be a nucleic acid capable of inhibiting expression(s) of (a) gene(s) encoding a COUP-TFI protein and/or a COUP-TFII protein. The nucleic acid may be an shRNA or an siRNA. Alternatively, the inhibitor may be a competitive inhibition mutant of a COUP-TFI protein or a COUP-TFII protein, wherein the competitive inhibition mutant includes a transcription activation domain and a DNA-binding domain of a COUP-TFI protein or a COUP-TFII protein. The competitive inhibition mutant may also comprise a COUP-TFI or COUP-TFII DNA-binding domain but lack a transcription inhibition domain. The NSPC is derived from a stem cell that exists in vivo. The neuron that differentiates by any agent described above is a cholinergic neuron, a GABAergic neuron and a glutamatergic neuron, preferably an Isl-1-positive neuron, a DARPP-32-positive neuron or a Tbr1-positive neuron.

Also disclosed herein is a method for promoting neuronal differentiation of an NSPC that includes inhibiting function of a COUP-TFI protein and/or a COUP-TFII protein in the NSPC. The method may include inhibiting expression(s) of (a) gene(s) encoding a COUP-TFI protein and/or a COUP-TFII protein in the NSPC. In this method, a nucleic acid molecule capable of inhibiting expression of the gene is introduced into the NSPC. The nucleic acid molecule may be an shRNA or an siRNA. Further, a competitive inhibition mutant of a COUP-TFI protein or a COUP-TFII protein may be introduced into the NSPC. The competitive inhibition mutant may include a transcription activation domain and a DNA-binding domain of a COUP-TFI protein or a COUP-TFII protein. The NSPC may be derived from an induced pluripotent stem cell The neuron that differentiates by any method described above may be an Isl-1-positive neuron, a DARPP-32-positive neuron and a Tbr1-positive neuron. Furthermore, the differentiated neuron may be a cholinergic neuron, a GABAergic neuron and a glutamatergic neuron.

In another embodiment of the present invention, a pharmaceutical composition for use in a method of treatment includes an inhibitor of function of a COUP-TFI protein and/or a COUP-TFII protein. The inhibitor may be a nucleic acid capable of inhibiting expression(s) of (a) gene(s) encoding a COUP-TFI protein and/or a COUP-TFII protein. The nucleic acid may be an shRNA or an siRNA. Alternatively, the inhibitor may be a competitive inhibition mutant of a COUP-TFI protein or a COUP-TFII protein comprising a transcription activation domain and a DNA-binding domain of a COUP-TFI protein or a COUP-TFII protein. Alternatively, the inhibition mutant may comprise a COUP-TFI or COUP-TFII DNA-binding domain but lack a transcription inhibition domain. The inhibitor may inhibit the function of a COUP-TFI protein and/or a COUP-TFII protein in an NSPC in vivo. The NSPC may be derived from a stem cell. The neuron that differentiates by any pharmaceutical composition described above is a cholinergic neuron, a GABAergic neuron and a glutamatergic neuron. Furthermore, the differentiated neuron may be an Isl-1-positive neuron, a DARPP-32-positive neuron and a Tbr1-positive neuron. This pharmaceutical composition may be administered to a patient with brain ischemia, traumatic brain injury, Huntington's disease and Alzheimer's disease.

Also disclosed herein is a method for treating a neurological disorder that includes inhibiting function of a COUP-TFI protein and/or a COUP-TFII protein in a NSPC. The method includes inhibiting expression(s) of (a) gene(s) encoding a COUP-TFI protein and/or a COUP-TFII protein in the NSPC. In this method, a nucleic acid molecule capable of inhibiting expression of the gene may be introduced into the NSPC, and the nucleic acid molecule may be an shRNA or an siRNA. Further, a competitive inhibition mutant of a COUP-TFI protein or a COUP-TFII protein may be introduced into the NSPC. The competitive inhibition mutant may include a transcription activation domain and a DNA-binding domain of a COUP-TFI protein or a COUP-TFII protein or may include a COUP-TFI or COUP-TFII protein DNA-binding domain but lack a transcription inhibition domain. The NSPC may form a primary neurosphere. The neuron that differentiates by any method described above may be an Isl-1-positive neuron, a DARPP-32-positive neuron and a Tbr1-positive neuron. Furthermore, the differentiated neuron may be a cholinergic neuron, a GABAergic neuron and a glutamatergic neuron. The neurological disorder may be brain ischemia, traumatic brain injury, Huntington's disease and Alzheimer's disease.

### [Brief Description of Drawings]

[Fig. 1] Fig.1 is a graph showing the inhibition of the expression of the COUP-TF protein by knockdown of the Coup-tfI gene and/or Coup-tfII gene in an example according to the present invention.
[Fig. 2] Fig.2 is photographs showing the morphology of NSPCs in which the Coup-tfI gene and/or the Coup-tfII gene has been knocked down in an example according to the present invention.
[Fig. 3] Fig.3 is a graph showing the ratios of neurons, astrocytes and oligodendrocytes which appeared when differentiation of the NSPCs in which the Coup-tfI gene and/or the Coup-tfII gene had been knocked down was induced in an example according to the present invention.
[Fig. 4] Fig.4 is a graph showing the ratio of Isl-1 positive neurons that appeared when the NSPCs in which the Coup-tfI gene and the Coup-tfII gene had been knocked down were passaged and their differentiation was induced in an example according to the present invention.
[Fig. 5] Fig.5 is a graph showing the ratios of neurons, oligodendrocyte progenitor cells (OPCs) and other cells (Others) which differentiated from the brain cells, into which shRNA KDI+II had been introduced at E10.5, in the mouse brain at E16.5 in an example according to the present invention.
[Fig. 6] Fig.6 is a graph showing the ratios of neurons, astrocytes and other cells (Others) which differentiated from the brain cells, into which shRNA KDI+II had been introduced at E12.5, in the mouse brain at P20 in an Example according to the present invention.
[Fig. 7] Fig.7 is graphs showing the ratios of Isl-1 positive neurons, DARPP-32 positive neurons and Tbr1 positive neurons, which appeared when the Coup-tfI gene and the Coup-tfII gene had been knocked down in vivo in an example according to the present invention.
[Fig. 8] Fig.8 is a schematic diagram showing the structures of the fusion proteins to be used in the experiment of the competitive inhibition of the DNA binding function of the COUP-TF protein in an example according to the present invention.
[Fig. 9] Fig.9 is a graph showing the ratios of neurons that appeared when the NSPCs, in which the DNA binding function of the COUP-TF protein was competitively inhibited, were induced to differentiate in an example according to the present invention.

### [Description of Embodiments]

Embodiments of the present invention accomplished based on the above-described findings are hereinafter described in detail by giving reference Examples. Where there is no particular explanations in embodiments or Examples, methods described in standard sets of protocols such as J. Sambrook, E. F. Fritsch & T. Maniatis (Ed.) Molecular cloning, a laboratory manual (3rd edition) Cold Spring Harbor Press, Cold Spring Harbor, New York (2001); F. M. Ausubel, R. Brent, R. E. Kingston, D. D. Moore, J.G. Seidman, J. A. Smith, K. Struhl (Ed.) Current Protocols in Molecular Biology, John Wiley & Sons Ltd., or their modified/changed methods are used. When using a commercial reagent kit and/or a measuring apparatus, protocols attached to them are used unless otherwise explained.

The object, characteristics, advantages of the present invention as well as the idea thereof are apparent to those skilled in the art from the descriptions given herein, and the present invention can be easily conducted by those skilled in the art based on the descriptions given herein. It should be understood that the embodiments and specific examples of the invention described herein below are to be taken as preferred examples of the present invention. These descriptions are only for illustrative and explanatory purposes and are not intended to limit the present inventions to these embodiments or examples. It is further apparent to those skilled in the art that various changes and modifications may be made based on the descriptions given herein within the intent and scope of the present invention disclosed herein.

### <Neural stem/progenitor cells (NSPCs)>

The agent for use in a method of treatment according to the present invention is used for promoting neuronal differentiation of NSPCs and contains an inhibitor of function of COUP-TFI protein and/or COUP-TFII protein. In this specification, promotion of neuronal differentiation of NSPCs means a phenomenon in which the ratio of glia decreased and the ratio of neurons increased in differentiation of the NSPCs by administering the agent for promoting neuronal differentiation. The NSPCs are cells present in vivo.

As the NSPCs are present in vivo in the cerebral cortex during embryonic/fetal stages as well as in the hippocampus and lateral ventricle during adult stage of human or non-human vertebrates, the agent for promoting neuronal differentiation may be administered to an individual animal to promote neuronal differentiation of the NSPCs in vivo.

NSPCs in culture may be prepared by isolating and culturing the NSPCs in vivo. Alternatively, they may be prepared by differentiating induced pluripotent stem cells (iPS cells) into NSPCs. The method for inducing differentiation of iPS cells is disclosed in Japanese Patent Application Laid-open Publication No.2002-291469 in detail.

To improve differentiation potential into neurons when inducing differentiation, embryoid bodies (EBs) can be formed from iPS cells in advance of differentiation into NSPCs. The agent can then be administered to NSPCs under the culturing condition. The formation of EBs can be achieved by, for example, culturing iPS cells in the presence of retinoic acid or Noggin protein. The retinoic acid can be added at a low level (10⁻⁹ M to 10⁻⁶ M) to the medium. In the case of Noggin protein, the Xenopus noggin gene may be introduced into cultured mammalian cells to express the Noggin protein transiently and then the culture supernatant may be recovered and used as it is (1 to 50% (v/v)), or alternatively, recombinant Noggin protein (final concentration of approx. 1 ug/ml) may be used.

By dissociating EBs thus obtained and culturing them in suspension in a serum-free culture medium supplemented with FGF-2 (10 to 100 ng/ml) and sonic hedgehog protein (1 to 20 nM), NSPCs can differentiate and grow in a form of neurospheres. The addition of sonic hedgehog protein can improve the efficiency of the induction of differentiation from NSPCs into motoneuron precursor cells as well as the efficiency of growth, and indeed a subsequent culture allows cells to differentiate into motoneurons as well as into GABAergic neurons. Cells can be cultured in serum-free medium such as DMEM medium supplemented with glucose, glutamine, insulin, transferrin, progesterone, putrescine, selenium chloride, heparin and the like in addition to the above-mentioned supplements, or DMEM:F12 medium. Cells are typically cultured under the condition of 5% CO₂ at 35 to 40 °C for 7 to 9 days.

Neurospheres directly derived from EBs are referred to as primary neurospheres. The primary neurospheres can be dissociated and cultured under the same condition to form the neurospheres again, which are referred to as secondary neurospheres. Hereinafter neurospheres that underwent at least one passage under culturing condition are referred to as secondary neurospheres.

By culturing primary neurospheres in a general differentiation medium, mainly cholinergic neurons including motoneurons and GABAergic neurons are induced to differentiate. Differentiation can be performed by culturing cells in DMEM:F12 medium supplemented with glucose, glutamine, insulin, transferrin, progesterone, putrescine and selenium chloride, which corresponds to the medium for growing neural stem cells from which FGF and heparin are omitted. Sonic hedgehog protein may or may not be added in the medium. Cells can be cultured under the condition of 5% CO₂ at 35 to 40°C for 5 to 7 days. If secondary neurospheres are cultured under the same condition, they differentiate not only into neurons that are mainly cholinergic neurons including motoneurons and GABAergic neurons but also into glial cells.

### <Inhibitor of the function of COUP-TF protein>

COUP-TF protein (Coup-tfI and Coup-tfII are herein collectively referred to as Coup-tf in either case of a gene or a protein) may be derived from a vertebrate such as human and mouse. Although it has preferably the same origin as the NSPCs to be targeted, their origins may be different as long as the inhibitor can inhibit the function of the COUP-TF protein in the cell to be targeted.

The inhibitor may inhibit the activity of the COUP-TF protein directly, or it may inhibit the expression of the COUP-TF protein in a cell. The inhibitor to be used is a high molecular compound such as a nucleic acid or a protein.

Examples of the inhibitor nucleic acid include siRNAs, shRNAs and antisense RNAs capable of inhibiting the expression of the gene encoding the COUP-TF protein. It may be designed in any technique known to those skilled in the art, and its sequence, type of the nucleic acid (for example, it may include DNA or inosine), and modification are not particularly limited as long as it can function as an inhibitor. Further, it may inhibit the expression of the COUP-TF protein at either transcription level or translation level.

Examples of the inhibitor protein include a mutant COUP-TF protein that inhibits function of the COUP-TF wild-type protein competitively. For example, since the COUP-TF protein is a transcription regulator, forced expression of a recombinant mutant COUP-TF protein in cells, which has its DNA-binding domain and lacks its transcription inhibition domain, will inhibit binding of the endogenous COUP-TF protein to DNA competitively, resulting in inhibiting the function of the COUP-TF protein. For example, a DNA-binding domain of mouse COUP-TFI protein (NM_010151) is the region at position 86 to 149 of the amino acid sequence of SEQ ID NO.1, and that of COUP-TFII protein (NM_009697) is the region at position 80 to 144 of the amino acid sequence of SEQ ID NO.2; these regions may be used to construct a competitive inhibition mutant.

Further, as will be described in Examples, this competitive inhibition mutant may include a transcription activation domain. The transcription activation domain is not limited as long as it functions as an independent activation domain when bound with a DNA-binding domain of a heterologous protein, and any of known activation domains such as GAL4, Bicoid, c-Fos, B42 and VP16 may be used.

### <Agent for promoting neuronal differentiation and method for administering the agent>

The inhibitor of the function of the COUP-TF protein may be formulated as a neuronal differentiation promoting agent using any of pharmaceutically acceptable carrier, diluent, or excipient known to those skilled in the art. This agent may be used as a medical drug in vivo.

The disease to be targeted by the drug is not particularly limited as long as it is a defect requiring promotion of neuronal differentiation of the NSPCs for treatment in a human or non-human vertebrate, and examples of such defect include neurologic diseases and nerve injuries in central nervous system such as a cerebral infarction and a traumatic brain injury, brain ischemia, Huntington's disease and Alzheimer's disease as well as functional disorder by aging. The administration method and the dosage amount of the neuronal differentiation promoting agent for a patient can be appropriately chosen by those skilled in the art based on the administration purpose, the dosage form, the state of the patient etc.

It should be noted that the inhibitor may be used solely by itself or in a combination of multiple forms. For example, an inhibitor for COUP-TFI protein and an inhibitor for COUP-TFII protein may be used in a combination, or an inhibitor nucleic acid and an inhibitor protein for COUP-TF protein may be used in a combination.

Alternatively, an inhibitor which inhibits the functions of both the COUP-TFI protein and the COUP-TFII protein may be used. Such inhibitor may be designed by utilizing a conserved nucleotide sequence between the COUP-TF genes.

### [Example]

### Example 1

### <Knockdown of Coup-tf gene in NSPCs>

In this example, it is described that if a Coup-tf gene is knocked down in NSPCs, the cells preferentially differentiate into neurons when they are placed in a differentiation condition.

### (1) Culture of mouse ES cells and formation of embryoid bodies (EBs)

EB3 cells, which were obtained by inserting a Blasticidin-resistance gene at Oct3/4 locus of an ES cell line E14tg2a derived from a mouse strain 129/Ola to enable selection of undifferentiated ES cells, were cultured using Glasgow minimum essential medium (GMEM) supplemented with 10% FCS, non-essential amino acids, 1 mM sodium pyruvate, 0.1 mM 2-mercaptoehtanol and 1000 U/mL leukemia inhibitory factor (LIF) under the condition of 5% CO₂, 37°C.

Next, the ES cells were washed with PBS, dissociated by 0.25% trypsin-1 mM EDTA treatment, seeded in bacterial culture dishes at a concentration of 1 x 10⁵ cell/mL, and cultured in suspension for 4 to 8 days to allow formation of EBs using the culture supernatant as medium, which was obtained by transiently expressing the Xenopus Noggin gene in COS7 cells.

### (2) Isolation of NSPCs from EBs by selective culture

The EBs thus formed were treated with 0.25% trypsin-1mM EDTA solution and were dispersed into cells. The dispersed cells were seeded in bacterial culture dishes at a concentration of 5 x 10⁴ cells/mL in MHM medium, which is DMEM:F12(1:1) supplemented with glucose (0.6%), glutamate (2mM), insulin (25ug/mL), transferrin (100ug/mL), progesterone (20nM), putrescine (60uM), selenium chloride (30nM), FGF-2 (20ng/mL) and heparin (2ug/mL) and further added with mouse sonic hedgehog1 protein (5nM), and cultured in suspension for 7 to 9 days to allow formation of primary neurospheres.

### (3) Knockdown of Coup-tf gene

Sequences of shRNAs for knockdown of Coup-tfI gene and/or Coup-tfII gene were designed by using an online siRNA design program siDirect (http://design.mai.jp/). As for negative controls, pSilencer 2.1-U6 Negative Control (Ambion) as well as mutants of the shRNAs for Coup-tf knockdown, in which one, two or three bases were mutated were used. Their sequences are as follows.
KDI (for Coup-tfI):
   GATGCTGCCCACATCGAAATTCAAGAGATTTCGATGTGGGCAGCATC (SEQ ID NO.3)
KDII (for Coup-tfII KD) :
   GTCCCAGTGTGCTTTGGAATTCAAGAGATTGGAAAGCACACTGGGAC (SEQ ID NO.4)
KDI+II (for Coup-tf KD) :
   GTCGAGCGGCAAGCACTACTTCAAGAGAGTAGTGCTTGCCGCTCGAC (SEQ ID NO.5)
2.1-U6 :
   ACTACCGTTGTTATAGGTGTTCAAGAGACACCTATAACAACGGTAGT (SEQ ID NO.6)

To express these shRNAs under the control of histone H1 promoter, their complementary sequences were synthesized and their double strand DNAs were formed by annealing, each of which was inserted between BglII site and Xbal site of an Entry Vector for shRNA pENTR4-H1. Each of the inserted shRNAs and histone H1 promoter were recombined into pCS-RfA-EG, a lentivirus vector for expressing shRNA, by using Gateway system (Invitrogen). The recombinant lentivirus vectors were introduced into 293T cells along with pCMV-VSV-G-RSV-rev and pCAG-HIVgp by using FuGENE6 (Roche), and the resulting culture supernatants were recovered, from which recombinant lentiviruses were purified at high concentration by ultracentrifugation. It should be noted that the lentivirus vectors contain the GFP gene to be expressed constitutively in order to enable to identify infected cells.

The recovered lentiviruses were infected to the neurospheres at MOI25. Infected neurospheres were dispersed into single cells, and cultured in GMEM for 7 days to form secondary neurospheres. The neurospheres were subsequently passaged every 6 days.

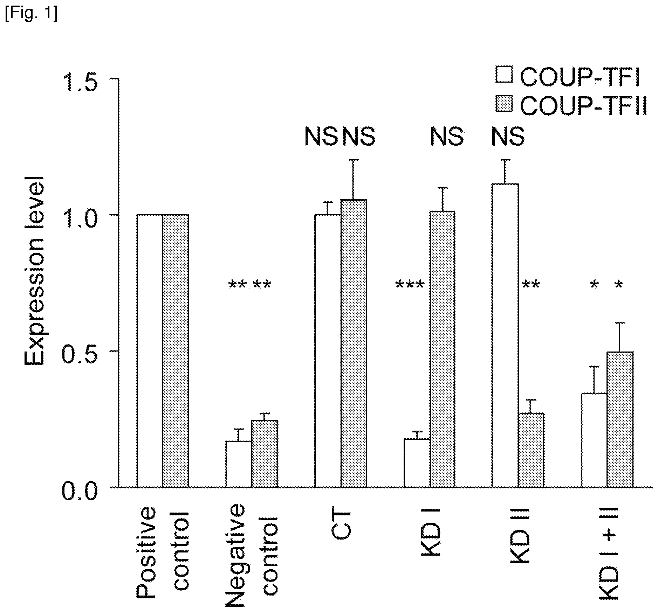

In order to confirm that the expression of the Coup-tf gene was inhibited by each of the shRNAs, the lentivirus vector expressing respective shRNA was introduced to Coup-tf expressing 293T cells to which the Coup-tf genes had been stably introduced to express both of the COUP-TF proteins and Western blotting was conducted on cell extracts of the shRNA-expressing cells. As for antibodies, anti-COUP-TFI antibody (mouse IgG, RRMX PP-H8132-00, 100-fold dilution) and anti-COUP-TFII antibody (mouse IgG, RRMX PP-H7147-00, 100-fold dilution) were used. Relative expression levels in respective cells of Negative control (wild-type 293T), CT (in which 2.1-U6 was introduced), KDI (in which KDI was introduced), KDII (in which KDII was introduced) and KDI+II (in which both KDI + KDII was introduced) were analyzed by t-test with normalizing the expression levels of each COUP-TF protein in Positive control (293T expressing Coup-tf) as 1.0, and plotted on a graph as shown in Fig.1 (the result of p<0.05, p<0.01 and p<0.001 relative to Positive control are labeled by *, ** and *** respectively. Similar labeling by using * with regard to the p value was applied to the following graphs as well). As shown in these results, each shRNA of KDI, KDII and KDI+II was capable of inhibiting the expression of the COUP-TFI protein, the COUP-TFII protein and both of them, respectively.

### (4) Analysis of knocked-down NSPCs

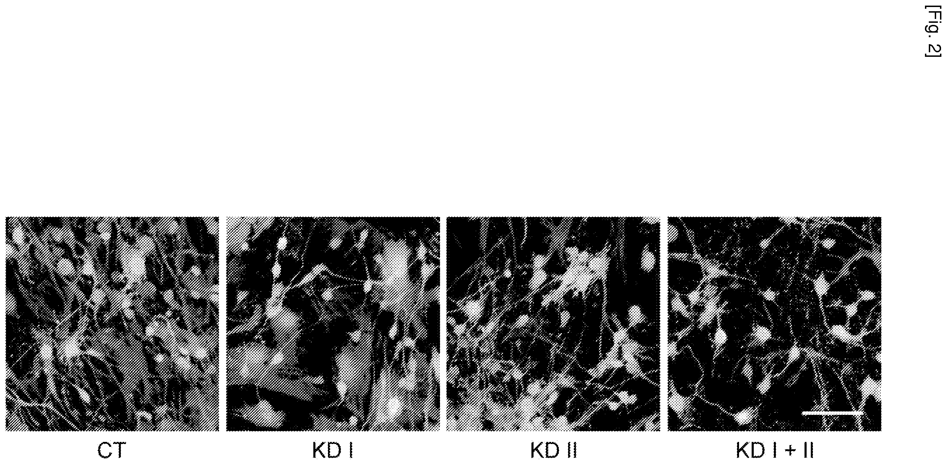

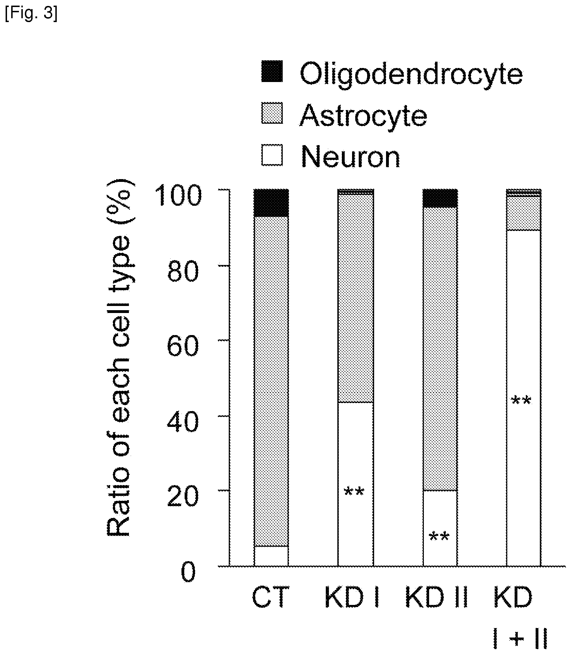

At each passage of neurospheres in which shRNAs had been introduced, a part of the neurospheres was dispersed by pipetting, seeded in a culture dish which was coated with poly-L-ornithin and filled with a differentiation medium, and grown in the presence of sonic hedgehog protein (5nM) for 5 to 7 days to differentiate. Then, the cells were immunostained with anti-beta III tubulin antibody used as a marker for neurons (mouse IgG, Sigma T8660, 1000-fold dilution), anti-GFAP antibody as a marker for astrocytes (rabbit IgG, DAKO Z0334, 400-fold dilution) and anti-04 antibody as a marker for oligodendrocytes (mouse IgM, Chemicon MAB345, 8000-fold dilution) to observe their cellular morphology and the stained cell types by using fluorescent microscopy (Fig.2). In each sample, cell numbers of neurons, astrocytes and oligodendrocytes were counted and ratios of each cell type were plotted on a graph (Fig.3).

In each of the analyses for the cellular morphology, the staining patterns and the ratios of each cell type, it was shown that the ratio of neurons increased by inhibiting expression of the COUP-TFI protein or the COUP-TFII protein, and further increased by inhibiting both the COUP-TFI and COUP-TFII proteins. It should be noted that even when LIF and BMP2, factors which facilitate differentiation into glial cells, were added to the culture medium with introduction of the shRNAs, similar results were obtained. Further, the efficiency of the formation of neurospheres did not change by inhibiting expression of the COUP-TFI protein or the COUP-TFII protein.

Thus, by inhibiting function of at least one of the COUP-TF proteins in NSPCs in vitro, the cells have come to differentiate into neurons preferentially when placed in the differentiating condition.

Further, by utilizing the expression of Isl-1, a marker for early differentiating neurons in forebrain, hindbrain and spinal cord, it was confirmed that while neurospheres lose potential to differentiate into neurons which are differentiated during early developmental processes of the central nervous system every passage, neurospheres in which the function of the COUP-TF protein is inhibited maintain the potential to differentiate into such early differentiating neurons.

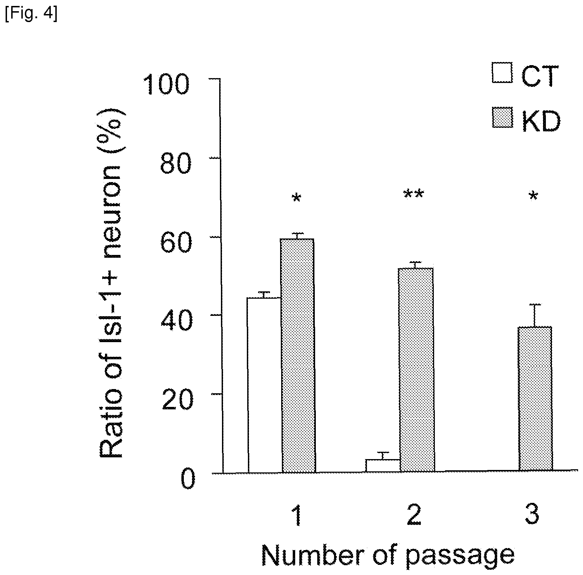

During passages of the neurospheres after the introduction of shRNAs, at each step of the first passage (to form the primary neurospheres), the second passage and the third passage, the cells were induced to differentiate and stained with anti-Isl-1 antibody (mouse IgG, DSHB 40.2D6, 200-fold dilution) to count the cell numbers of each cell type. Then the ratios of Isl-1 positive neurons against the number of total virus-infected neurons were calculated and plotted on a graph as shown in Fig.4. In the untreated cells (CT), the expression of Isl-1 have been almost lost by the third passage, whereas in the cells to which shRNA KDI+II were introduced, the expression of Isl-1 was maintained at almost the same level even after the third passage.

Thus, the change of the neuronal type due to the induction of differentiation can be suppressed by the inhibition of the function of the COUP-TF protein.

### Example 2

### <Knockdown of the Coup-tf gene in living mice>

In this Example, it is shown that during differentiation of NSPCs in a living mouse, knockdown of the Coup-tf gene induces neuronal differentiation.

### (1) Mice and viral infection

In this Example, the shRNA-containing lentivirus as described in Example 1 was microinjected into cerebral ventricles of mouse embryos in uteri of ICR mice at 10.5 days or 12.5 days after fertilization. For the microinjection at 10.5 days, VS40 and Vevo660 (VisualSonics) was used.

### (2) Analyses of mouse brain

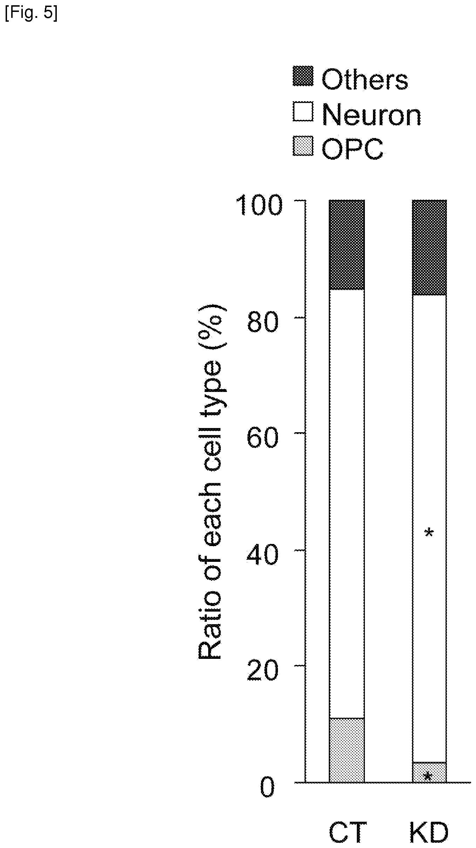

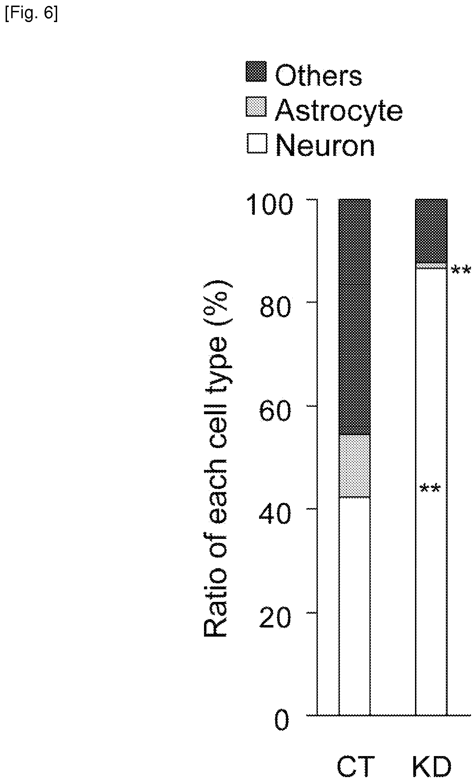

Immunohistochemical analyses were conducted at E16.5 for the brain of mice which had been infected with the shRNA-containing lentivirus at E10.5 (Fig.5), as well as at P20 for the brain of mice which had been infected at E12.5 (Fig.6). Cryosections of the brain were prepared and immunostaining was performed by standard protocols.

For the brain of 16.5-day embryo, anti-NeuN antibody was used as a marker for neurons; anti-Sox antibody and anti-Olig2 antibody were used as markers for oligodendrocyte precursor cells (OPCs), and double-positive cells were accounted as OPCs. Non-neurons other than OPCs were classified as "Others (other cells)".

As shown in Fig.5, the ratio of the OPCs in the cells to which shRNA KDI+II had been introduced (KD) decreased from 11% to 3.4% and the ratio of neurons increased from 73.8% to 80.4%, as compared with the negative control (CT) to which shRNA2.1-U6 had been introduced.

For the mice at P20, the anti-NeuN antibody was used as a marker for neurons, the anti-GFAP antibody was used as a marker for astrocytes, and non-neurons which were negative for the anti-GFAP antibody were classified as "Others".

As shown in Fig.6, the ratio of astrocytes in the cells to which shRNA KDI+II had been introduced (KD) decreased from 12.2% to 1.3% and the ratio of neurons increased from 42.2% to 86.5%, as compared with the negative control (CT) to which shRNA2.1-U6 had been introduced.

Thus, differentiation into neurons was promoted in vivo by inhibiting the function of the COUP-TF protein in the NSPCs.

### (3) Analyses of cell-type of the differentiated neurons

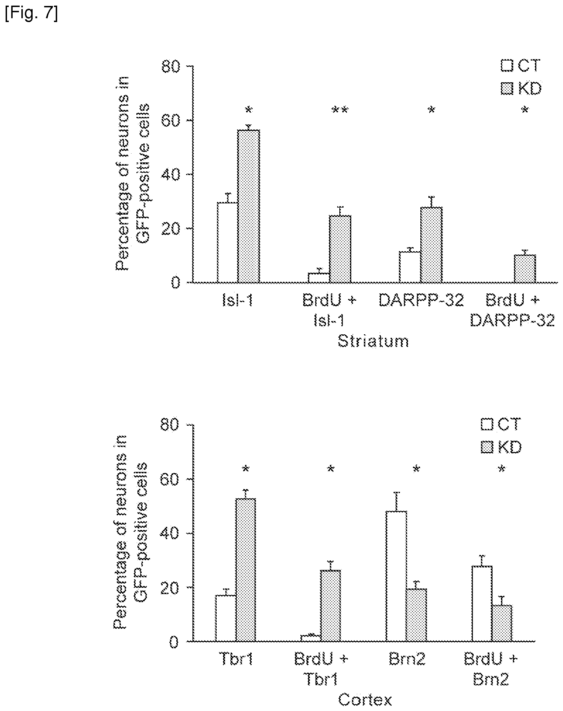

To determine the cell-types of the neurons that differentiated by inhibiting COUP-TF in vivo, immunohistochemical analyses were conducted at E16.5 for the brain of mice which had been infected with the shRNA-containing lentivirus at E10.5 (Fig.7), using anti-Isl-1 antibody, anti-DARPP-32 antibody (Chemicon, 1/2000 dilution), anti-Tbr1 antibody(obtained from Dr. R. F. Hevner (Univ. of Washington, 1/10000 dilution) or anti-Brn2 antibody(Santa Cruz Biotechnology, 1/500 dilution). Cells born at E15.5 were labeled by BrdU incorporation and immunohistochemistry for BrdU was performed using anti-BrdU antibody(Abcam, 1/100 dilution)after pretreating the sections in 1N HCl at 37°C for 30min to denature the DNA. Negative control used was the same as used in (2).

As shown in Fig.7, Isl-1 positive neurons and DARPP-32 positive neurons increased in striatum from 29.5 % to 56.4 % and from 11.5% to 27.7% as compared with the negative control (CT), respectively. The result of the double-staining with anti-BrdU antibody indicates that about half of the Isl-1 positive neurons and about one-third of the DARPP-32 positive neurons were born around E15.5. Tbr1 positive neurons also increased in cortex from 17.0% to 52.6% and about half of the Tbr1 positive neurons were born around E15.5 although they are normally barely generated after E15.5. Brn2 positive neurons, however, decreased from 48.1% to 19.2% in cortex, which might be sacrificed to the increase of Tbr1 positive neurons. It should be noted that the Isl-1 positive neurons in striatum are common precursors of cholinergic and GABAergic neurons and differentiated cholinergic neurons including motoneurons, the DARPP-32 positive neurons in striatum are GABAergic neurons, and the Tbr1 positive neurons in cortex are glutamatergic neurons. This differentiation pattern is consistent with the fact that neurospheres are induced to differentiate mainly into cholinergic neurons including motoneurons and GABAergic neurons in a general differentiation medium in vitro.

Thus the neurons that have differentiated by inhibiting the function of the COUP-TF protein in the NSPCs are at least partly Isl-1-positive, DARPP-32-positive or Tbr1-positive neurons, which should be cholinergic neurons, GABAergic neurons or glutamatergic neurons.

### Example 3

### <Competitive inhibition of DNA binding function of the COUP-TF protein in NSPCs>

In this Example, it is shown that neuronal differentiation can be promoted by competitive inhibition of the DNA binding function of the COUP-TF protein in NSPCs.

### (1) Fusion proteins

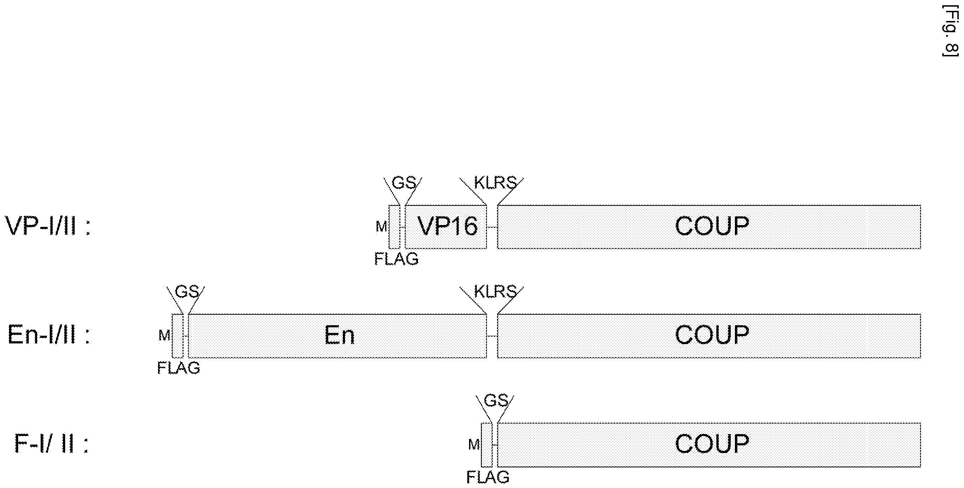

As for the fusion proteins to be used in this Example, either one of the DNA-binding domain at the N-terminal of the COUP-TFI protein (the amino acid sequence at position 2 to 403 of SEQ ID NO.1) or the DNA-binding domain at the N-terminal of the COUP-TFII protein (the amino acid sequence at position 2 to 394 of SEQ ID NO.2) was fused with either one of the transcription activation domain of VP16 (the amino acid sequence at position 413 to 490 of SEQ ID NO.7 <P06492>) or the transcription repression domain of Drosophila Engrailed (En) protein (the amino acid sequence at position 2 to 295 of SEQ ID NO.8 <NM 078976>) to construct either a dominant positive mutant (hereinafter referred to as VP-1 or VP-II) or a dominant negative mutant (hereinafter referred to as EnI or EnII) and used as respective mixtures (the mixed proteins are hereinafter referred to as VP-I/II and EnI/II, respectively). These fusion proteins do not have the transcription regulatory domain which is present at the N-terminus of the COUP-TF protein. As for control, COUP-TFI/II (a mixture of wild-type COUP-TFI and COUP-TFII proteins as well as F-I/II (a mixture of wild-type COUP-TFI and wild-type COUP-TFII, both of which are bound with a flag-tag (DYKDDDDK: SEQ ID NO.9)) were used. Briefly, VP-1, VP-II, Enl and EnI have a structure represented as Met-FLAG-GlySer-VP/En-LysLeuArgSer-COUP, and F-I and F-II have a structure represented by Met-FLAG-GlySer-COUP (VP/EN indicates either one of VP-1, VP-II, EnI or EnII; and COUP indicates the DNA-binding domain of COUP-TFI or COUP-TFII). Schematic diagrams of these fusion proteins are shown in Fig.8.

Recombinant DNAs encoding these fusion proteins were inserted instead of the shRNA in Example 1 into the lentivirus vector, and recombinant lentiviruses were produced by using 293T cells.

### (2) Expression and analyses of the fusion proteins in NSPCs

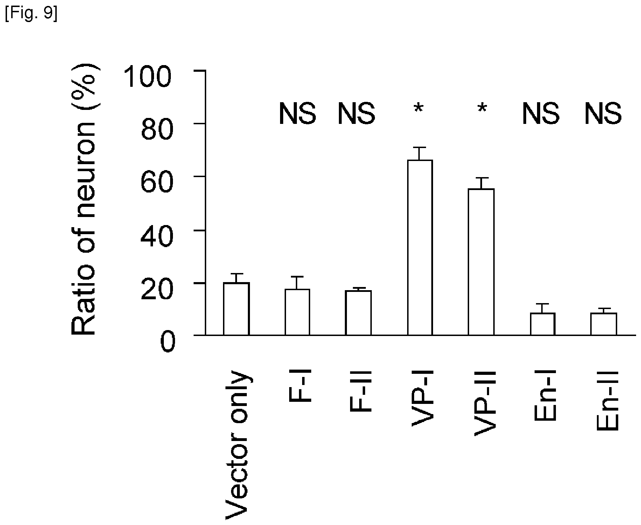

Like Example 1, by using the NSPCs derived from ES cells, primary neurospheres were infected with lentiviruses having the recombinant genes encoding the fusion proteins, and the cells were allowed to differentiate after the third passage, and ratios of neurons in the total cells were counted. The results were plotted on a graph as shown in Fig.9.

Among the fusion proteins, only the dominant positive mutants (VP-1/II) promoted differentiation into neurons. This suggests that the COUP-TF protein functions as a repressor.

Thus, not only the inhibition of expression by using shRNAs but also the competitive inhibition against DNA binding by using the fusion protein can inhibit the function of the COUP-TF protein in the neural stem / progenitor cell, thereby promoting differentiation to neurons.

### [Industrial applicability]

The present invention enables to provide agents for promoting neuronal differentiation of NSPCs and methods therefor.

## Claims

1. An inhibitor for use in a method of treatment of the human or animal body wherein neuronal differentiation of a neural stem/progenitor cell is promoted, wherein the neural stem/progenitor cell exists *in vivo,* wherein the differentiated neuron is a cholinergic neuron, a GABAergic neuron, or a glutamatergic neuron, wherein the inhibitor inhibits expression of one or more genes encoding a COUP-TFI protein and/or a COUP-TFII protein, wherein the inhibitor is a nucleic acid capable of inhibiting expression of the COUP-TFI or COUP-TFII gene.

2. An inhibitor for use according to claim 1, wherein the nucleic acid is a sh RNA or an si RNA.

3. An inhibitor for use in a method of treatment of the human or animal body wherein neuronal differentiation of a neural stem/progenitor cell is promoted, wherein the neural stem/progenitor cell exists *in vivo,* wherein the differentiated neuron is a cholinergic neuron, a GABAergic neuron, or a glutamatergic neuron, and wherein the inhibitor is a competitive inhibition mutant of a COUP-TFI protein or a COUP-TFII protein, and wherein the competitive inhibition mutant comprises a DNA-binding domain of a COUP-TFI protein or a COUP-TFII protein and lacks a transcription inhibition domain.

4. An inhibitor for use according to any previous claim, wherein the differentiated neuron is Isl-1-positive, DARPP-32-positive or Tbr1-positive.

5. An inhibitor for use according to any previous claim, wherein the method of treatment is of a neurological disorder, which is preferably a disease selected from the group consisting of brain ischemia, traumatic brain injury, Huntington's disease and Alzheimer's disease.

6. A pharmaceutical composition for use in a method of treatment of a human or animal body wherein neuronal differentiation of a neural stem/progenitor cell is promoted, wherein the neural stem/progenitor cell exists *in vivo,* wherein the differentiated neuron is a cholinergic neuron, a GABAergic neuron, or a glutamatergic neuron, and wherein the pharmaceutical composition comprises a nucleic acid inhibitor which inhibits expression of one or more genes encoding a COUP-TFI protein and/or a COUP-TFII protein or is a competitive inhibition mutant of a COUP-TFI protein or a COUP-TFII protein comprising a DNA-binding domain of a COUP-TFI or COUP-TFII protein and lacks a transcription inhabitation domain.

7. A pharmaceutical composition for use according to claim 6, wherein the method of treatment is of a disease selected from the group consisting of brain ischemia, traumatic brain injury, Huntington's disease and Alzheimer's disease.

## Patentansprüche

1. Hemmer zur Verwendung in einem Verfahren zum Behandeln des menschlichen oder tierischen Körpers, wobei neuronale Differenzierung einer neuralen Stamm-/Progenitorzelle gefördert wird, wobei die neurale Stamm-/Progenitorzelle *in vivo* vorliegt, wobei das differenzierte Neuron ein cholinerges Neuron, ein GABAerges Neuron oder ein glutamaterges Neuron ist, wobei der Hemmer die Expression eines oder mehrerer Gene, das/die für ein COUP-TFI-Protein und/oder ein COUP-TFII-Protein codiert/codieren, hemmt, wobei der Hemmer eine Nukleinsäure ist, die in der Lage ist, Expression des COUP-TFI- oder COUP-TFII-Gens zu hemmen.

2. Hemmer zur Verwendung nach Anspruch 1, wobei die Nukleinsäure eine shRNA oder eine siRNA ist.

3. Hemmer zur Verwendung in einem Verfahren zum Behandeln des menschlichen oder tierischen Körpers, wobei neuronale Differenzierung einer neuralen Stamm-/Progenitorzelle gefördert wird, wobei die neurale Stamm-/Progenitorzelle *in vivo* vorliegt, wobei das differenzierte Neuron ein cholinerges Neuron, ein GABAerges Neuron oder ein glutamaterges Neuron ist und wobei der Hemmer eine kompetitive Hemmungsmutante eines COUP-TFI-Proteins oder eines COUP-TFII-Proteins ist, und wobei die kompetitive Hemmungsmutante eine DNA-Bindungsdomäne eines COUP-TFI-Proteins oder eines COUP-TFII-Proteins umfasst und dieser eine Transkriptions-Hemmungsdomäne fehlt.

4. Hemmer zur Verwendung nach einem der vorhergehenden Ansprüche, wobei das differenzierte Neuron Isl-1-positiv, DARPP-32-positiv oder Tbr1-positiv ist.

5. Hemmer zur Verwendung nach einem der vorhergehenden Ansprüche, wobei das Verfahren zum Behandeln für eine neurologische Störung vorgesehen ist, die vorzugsweise eine Krankheit ist, ausgewählt aus der Gruppe bestehend aus Hirnischämie, Schädel-Hirn-Trauma, Huntington-Krankheit und Alzheimer-Krankheit.

6. Pharmazeutische Zusammensetzung zur Verwendung in einem Verfahren zum Behandeln eines menschlichen oder tierischen Körpers, wobei neuronale Differenzierung einer neuralen Stamm-/Progenitorzelle gefördert wird, wobei die neurale Stamm-/Progenitorzelle *in vivo* vorliegt, wobei das differenzierte Neuron ein cholinerges Neuron, ein GABAerges Neuron oder ein glutamaterges Neuron ist, und wobei die pharmazeutische Zusammensetzung einen Nukleinsäurehemmer umfasst, der Expression eines oder mehrerer Gene, das/die für ein COUP-TFI-Protein und/oder ein COUP-TFII-Protein codiert/codieren, hemmt, oder eine kompetitive Hemmungsmutante eines COUP-TFI-Proteins oder eines COUP-TFII-Proteins, umfassend eine DNA-Bindungsdomäne eines COUP-TFI- oder eines COUP-TFII-Proteins, ist und dieser eine Transkriptions-Hemmungsdomäne fehlt.

7. Pharmazeutische Zusammensetzung zur Verwendung nach Anspruch 6, wobei das Verfahren zum Behandeln für eine Krankheit vorgesehen ist, die ausgewählt ist aus der Gruppe bestehend aus Hirnischämie, Schädel-Hirn-Trauma, Huntington-Krankheit und Alzheimer-Krankheit.

## Revendications

1. Inhibiteur à utiliser dans un procédé de traitement du corps humain ou animal, dans lequel une différenciation neuronale d'une cellule souche/progénitrice neurale est favorisée, dans lequel la cellule souche/progénitrice neurale existe *in vivo,* dans lequel le neurone différencié est un neurone cholinergique, un neurone GABAergique ou un neurone glutamatergique, dans lequel l'inhibiteur inhibe l'expression d'un ou plusieurs gènes codant pour une protéine COUP-TFI et/ou une protéine COUP-TFII, dans lequel l'inhibiteur est un acide nucléique capable d'inhiber l'expression du gène COUP-TFI ou COUP-TFII.

2. Inhibiteur à utiliser selon la revendication 1, dans lequel l'acide nucléique est un ARNsh ou un ARNsi.

3. Inhibiteur à utiliser dans un procédé de traitement du corps humain ou animal, dans lequel une différenciation neuronale d'une cellule souche/progénitrice neurale est favorisée, dans lequel la cellule souche/progénitrice neurale existe *in vivo,* dans lequel le neurone différencié est un neurone cholinergique, un neurone GABAergique ou un neurone glutamatergique, et dans lequel l'inhibiteur est un mutant d'inhibition compétitive d'une protéine COUP-TFI ou d'une protéine COUP-TFII, et dans lequel le mutant d'inhibition compétitive comprend un domaine de liaison à l'ADN d'une protéine COUP-TFI ou d'une protéine COUP-TFII et n'a pas de domaine d'inhibition de la transcription.

4. Inhibiteur à utiliser selon l'une quelconque des revendications précédentes, dans lequel le neurone différencié est positif à Isl-1, positif à DARPP-32 ou positif à Tbr1.

5. Inhibiteur à utiliser selon l'une quelconque des revendications précédentes, dans lequel le procédé de traitement concerne un trouble neurologique, qui est de préférence une maladie sélectionnée dans le groupe constitué par l'ischémie cérébrale, la lésion cérébrale traumatique, la maladie de Huntington et la maladie d'Alzheimer.

6. Composition pharmaceutique à utiliser dans un procédé de traitement d'un corps humain ou animal, dans laquelle une différenciation neuronale d'une cellule souche/progénitrice neurale est favorisée, dans laquelle la cellule souche/progénitrice neurale existe *in vivo,* dans laquelle le neurone différencié est un neurone cholinergique, un neurone GABAergique ou un neurone glutamatergique, et dans laquelle la composition pharmaceutique comprend un inhibiteur d'acide nucléique qui inhibe l'expression d'un ou plusieurs gènes codant pour une protéine COUP-TFI et/ou une protéine COUP-TFII ou est un mutant d'inhibition compétitive d'une protéine COUP-TFI ou d'une protéine COUP-TFII comprenant un domaine de liaison à l'ADN d'une protéine COUP-TFI ou COUP-TFII et n'a pas de domaine d'inhibition de la transcription.

7. Composition pharmaceutique à utiliser selon la revendication 6, dans laquelle le procédé de traitement concerne une maladie sélectionnée dans le groupe constitué par l'ischémie cérébrale, la lésion cérébrale traumatique, la maladie de Huntington et la maladie d'Alzheimer.


### SEQUENCE LISTING

<110> Keio University
<120> Agent for promoting neuronal differentiation and method therefor
<130> ICC00103EP
<160> 9
<170> PatentIn version 3.5
<210> 1
   <211> 420
   <212> PRT
   <213> Mus musculus
<400> 1
   Met Ala Met Val Val Ser Ser Trp Arg Asp Pro Gln Asp Asp Val Ala
   1               5                   10                  15
   Gly Gly Asn Pro Gly Gly Pro Asn Pro Ala Ala Gln Ala Ala Arg Gly
               20                  25                  30
   Gly Gly Gly Gly Glu Gln Gln Gln Ala Gly Ser Gly Ala Pro His Thr
           35                  40                  45
   Pro Gln Thr Pro Gly Gln Pro Gly Ala Pro Ala Thr Pro Gly Thr Ala
       50                  55                  60
   Gly Asp Lys Gly Gln Gly Pro Pro Gly Ser Gly Gln Ser Gln Gln His
   65                  70                  75                  80
   Ile Glu Cys Val Val Cys Gly Asp Lys Ser Ser Gly Lys His Tyr Gly
                   85                  90                  95
   Gln Phe Thr Cys Glu Gly Cys Lys Ser Phe Phe Lys Arg Ser Val Arg
               100                 105                 110
   Arg Asn Leu Thr Tyr Thr Cys Arg Ala Asn Arg Asn Cys Pro Ile Asp
           115                 120                 125
   Gln His His Arg Asn Gln Cys Gln Tyr Cys Arg Leu Lys Lys Cys Leu
       130                 135                 140
   Lys Val Gly Met Arg Arg Glu Ala Val Gln Arg Gly Arg Met Pro Pro
   145                 150                 155                 160
   Thr Gln Pro Asn Pro Gly Gln Tyr Ala Leu Thr Asn Gly Asp Pro Leu
                   165                 170                 175
   Asn Gly His Cys Tyr Leu Ser Gly Tyr Ile Ser Leu Leu Leu Arg Ala
               180                 185                 190
   Glu Pro Tyr Pro Thr Ser Arg Tyr Gly Ser Gln Cys Met Gln Pro Asn
           195                 200                 205
   Asn Ile Met Gly Ile Glu Asn Ile Cys Glu Leu Ala Ala Arg Leu Leu
       210                 215                 220
   Phe Ser Ala Val Glu Trp Ala Arg Asn Ile Pro Phe Phe Pro Asp Leu
   225                 230                 235                 240
   Gln Ile Thr Asp Gln Val Ser Leu Leu Arg Leu Thr Trp Ser Glu Leu
                   245                 250                 255
   Phe Val Leu Asn Ala Ala Gln Cys Ser Met Pro Leu His Val Ala Pro
               260                 265                 270
   Leu Leu Ala Ala Ala Gly Leu His Ala Ser Pro Met Ser Ala Asp Arg
           275                 280                 285
   Val Val Ala Phe Met Asp His Ile Arg Ile Phe Gln Glu Gln Val Glu
       290                 295                 300
   Lys Leu Lys Ala Leu His Val Asp Ser Ala Glu Tyr Ser Cys Leu Lys
   305                 310                 315                 320
   Ala Ile Val Leu Phe Thr Ser Asp Ala Cys Gly Leu Ser Asp Ala Ala
                   325                 330                 335
   His Ile Glu Ser Leu Gln Glu Lys Ser Gln Cys Ala Leu Glu Glu Tyr
               340                 345                 350
   Val Arg Ser Gln Tyr Pro Asn Gln Pro Ser Arg Phe Gly Lys Leu Leu
           355                 360                 365
   Leu Arg Leu Pro Ser Leu Arg Thr Val Ser Ser Ser Val Ile Glu Gln
       370                 375                 380
   Leu Phe Phe Val Arg Leu Val Gly Lys Thr Pro Ile Glu Thr Leu Ile
   385                 390                 395                 400
   Arg Asp Met Leu Leu Ser Gly Ser Ser Phe Asn Trp Pro Tyr Met Ser
                   405                 410                 415
   Ile Gln Cys Ser
               420
<210> 2
   <211> 414
   <212> PRT
   <213> Mus musculus
<400> 2
   Met Ala Met Val Val Ser Thr Trp Arg Asp Pro Gln Asp Glu Val Pro
   1               5                   10                  15
   Gly Ser Gln Gly Ser Gln Ala Ser Gln Ala Pro Pro Val Pro Gly Pro
               20                  25                  30
   Pro Pro Gly Ala Pro His Thr Pro Gln Thr Pro Gly Gln Gly Gly Pro
           35                  40                  45
   Ala Ser Thr Pro Ala Gln Thr Ala Ala Gly Gly Gln Gly Gly Pro Gly
       50                  55                  60
   Gly Pro Gly Ser Asp Lys Gln Gln Gln Gln Gln His Ile Glu Cys Val
   65                  70                  75                  80
   Val Cys Gly Asp Lys Ser Ser Gly Lys His Tyr Gly Gln Phe Thr Cys
                   85                  90                  95
   Glu Gly Cys Lys Ser Phe Phe Lys Arg Ser Val Arg Arg Asn Leu Ser
               100                 105                 110
   Tyr Thr Cys Arg Ala Asn Arg Asn Cys Pro Ile Asp Gln His His Arg
           115                 120                 125
   Asn Gln Cys Gln Tyr Cys Arg Leu Lys Lys Cys Leu Lys Val Gly Met
       130                 135                 140
   Arg Arg Glu Ala Val Gln Arg Gly Arg Met Pro Pro Thr Gln Pro Thr
   145                 150                 155                 160
   His Gly Gln Phe Ala Leu Thr Asn Gly Asp Pro Leu Asn Cys His Ser
                   165                 170                 175
   Tyr Leu Ser Gly Tyr Ile Ser Leu Leu Leu Arg Ala Glu Pro Tyr Pro
               180                 185                 190
   Thr Ser Arg Phe Gly Ser Gln Cys Met Gln Pro Asn Asn Ile Met Gly
           195                 200                 205
   Ile Glu Asn Ile Cys Glu Leu Ala Ala Arg Met Leu Phe Ser Ala Val
       210                 215                 220
   Glu Trp Ala Arg Asn Ile Pro Phe Phe Pro Asp Leu Gln Ile Thr Asp
   225                 230                 235                 240
   Gln Val Ala Leu Leu Arg Leu Thr Trp Ser Glu Leu Phe Val Leu Asn
                   245                 250                 255
   Ala Ala Gln Cys Ser Met Pro Leu His Val Ala Pro Leu Leu Ala Ala
               260                 265                 270
   Ala Gly Leu His Ala Ser Pro Met Ser Ala Asp Arg Val Val Ala Phe
           275                 280                 285
   Met Asp His Ile Arg Ile Phe Gln Glu Gln Val Glu Lys Leu Lys Ala
           290                 295                 300
   Leu His Val Asp Ser Ala Glu Tyr Ser Cys Leu Lys Ala Ile Val Leu
   305                 310                 315                 320
   Phe Thr Ser Asp Ala Cys Gly Leu Ser Asp Val Ala His Val Glu Ser
                   325                 330                 335
   Leu Gln Glu Lys Ser Gln Cys Ala Leu Glu Glu Tyr Val Arg Ser Gln
               340                 345                 350
   Tyr Pro Asn Gln Pro Thr Arg Phe Gly Lys Leu Leu Leu Arg Leu Pro
           355                 360                 365
   Ser Leu Arg Thr Val Ser Ser Ser Val Ile Glu Gln Leu Phe Phe Val
       370                 375                 380
   Arg Leu Val Gly Lys Thr Pro Ile Glu Thr Leu Ile Arg Asp Met Leu
   385                 390                 395                 400
   Leu Ser Gly Ser Ser Phe Asn Trp Pro Tyr Met Ala Ile Gln
                   405                 410
<210> 3
   <211> 47
   <212> DNA
   <213> Artificial Sequence
<220>
   <223> primer
<400> 3
   gatgctgccc acatcgaaat tcaagagatt tcgatgtggg cagcatc 47
<210> 4
   <211> 47
   <212> DNA
   <213> Artificial Sequence
<220>
   <223> primer
<400> 4
   gatgctgccc acatcgaaat tcaagagatt tcgatgtggg cagcatc 47
<210> 5
   <211> 47
   <212> DNA
   <213> Artificial Sequence
<220>
   <223> primer
<400> 5
   gtcgagcggc aagcactact tcaagagagt agtgcttgcc gctcgac 47
<210> 6
   <211> 47
   <212> DNA
   <213> Artificial Sequence
<220>
   <223> primer
<400> 6
   actaccgttg ttataggtgt tcaagagaca cctataacaa cggtagt 47
<210> 7
   <211> 490
   <212> PRT
   <213> herpes simplex virus 7
<400> 7
   Met Asp Leu Leu Val Asp Glu Leu Phe Ala Asp Met Asn Ala Asp Gly
   1               5                   10                  15
   Ala Ser Pro Pro Pro Pro Arg Pro Ala Gly Gly Pro Lys Asn Thr Pro
               20                  25                  30
   Ala Ala Pro Pro Leu Tyr Ala Thr Gly Arg Leu Ser Gln Ala Gln Leu
           35                  40                  45
   Met Pro Ser Pro Pro Met Pro Val Pro Pro Ala Ala Leu Phe Asn Arg
       50                  55                  60
   Leu Leu Asp Asp Leu Gly Phe Ser Ala Gly Pro Ala Leu Cys Thr Met
   65                  70                  75                  80
   Leu Asp Thr Trp Asn Glu Asp Leu Phe Ser Ala Leu Pro Thr Asn Ala
                   85                  90                  95
   Asp Leu Tyr Arg Glu Cys Lys Phe Leu Ser Thr Leu Pro Ser Asp Val
               100                 105                 110
   Val Glu Trp Gly Asp Ala Tyr Val Pro Glu Arg Thr Gln Ile Asp Ile
           115                 120                 125
   Arg Ala His Gly Asp Val Ala Phe Pro Thr Leu Pro Ala Thr Arg Asp
       130                 135                 140
   Gly Leu Gly Leu Tyr Tyr Glu Ala Leu Ser Arg Phe Phe His Ala Glu
   145                 150                 155                 160
   Leu Arg Ala Arg Glu Glu Ser Tyr Arg Thr Val Leu Ala Asn Phe Cys
                   165                 170                 175
   Ser Ala Leu Tyr Arg Tyr Leu Arg Ala Ser Val Arg Gln Leu His Arg
               180                 185                 190
   Gln Ala His Met Arg Gly Arg Asp Arg Asp Leu Gly Glu Met Leu Arg
           195                 200                 205
   Ala Thr Ile Ala Asp Arg Tyr Tyr Arg Glu Thr Ala Arg Leu Ala Arg
       210                 215                 220
   Val Leu Phe Leu His Leu Tyr Leu Phe Leu Thr Arg Glu Ile Leu Trp
   225                 230                 235                 240
   Ala Ala Tyr Ala Glu Gln Met Met Arg Pro Asp Leu Phe Asp Cys Leu
                   245                 250                 255
   Cys Cys Asp Leu Glu Ser Trp Arg Gln Leu Ala Gly Leu Phe Gln Pro
               260                 265                 270
   Phe Met Phe Val Asn Gly Ala Leu Thr Val Arg Gly Val Pro Ile Glu
           275                 280                 285
   Ala Arg Arg Leu Arg Glu Leu Asn His Ile Arg Glu His Leu Asn Leu
       290                 295                 300
   Pro Leu Val Arg Ser Ala Ala Thr Glu Glu Pro Gly Ala Pro Leu Thr
   305                 310                 315                 320
   Thr Pro Pro Thr Leu His Gly Asn Gln Ala Arg Ala Ser Gly Tyr Phe
                   325                 330                 335
   Met Val Leu Ile Arg Ala Lys Leu Asp Ser Tyr Ser Ser Phe Thr Thr
               340                 345                 350
   Ser Pro Ser Glu Ala Val Met Arg Glu His Ala Tyr Ser Arg Ala Arg
           355                 360                 365
   Thr Lys Asn Asn Tyr Gly Ser Thr Ile Glu Gly Leu Leu Asp Leu Pro
       370                 375                 380
   Asp Asp Asp Ala Pro Glu Glu Ala Gly Leu Ala Ala Pro Arg Leu Ser
   385                 390                 395                 400
   Phe Leu Pro Ala Gly His Thr Arg Arg Leu Ser Thr Ala Pro Pro Thr
                   405                 410                 415
   Asp Val Ser Leu Gly Asp Glu Leu His Leu Asp Gly Glu Asp Val Ala
               420                 425                 430
   Met Ala His Ala Asp Ala Leu Asp Asp Phe Asp Leu Asp Met Leu Gly
           435                 440                 445
   Asp Gly Asp Ser Pro Gly Pro Gly Phe Thr Pro His Asp Ser Ala Pro
       450                 455                 460
   Tyr Gly Ala Leu Asp Met Ala Asp Phe Glu Phe Glu Gln Met Phe Thr
   465                 470                 475                 480
   Asp Ala Leu Gly Ile Asp Glu Tyr Gly Gly
   485 490
<210> 8
   <211> 552
   <212> PRT
   <213> Drosophila melanogaster
<400> 8
   Met Ala Leu Glu Asp Arg Cys Ser Pro Gln Ser Ala Pro Ser Pro Ile
   1               5                   10                  15
   Thr Leu Gln Met Gln His Leu His His Gln Gln Gln Gln Gln Gln Gln
               20                  25                  30
   Gln Gln Gln Gln Met Gln His Leu His Gln Leu Gln Gln Leu Gln Gln
           35                  40                  45
   Leu His Gln Gln Gln Leu Ala Ala Gly Val Phe His His Pro Ala Met
       50                  55                  60
   Ala Phe Asp Ala Ala Ala Ala Ala Ala Ala Ala Ala Ala Ala Ala Ala
   65                  70                  75                  80
   Ala His Ala His Ala Ala Ala Leu Gln Gln Arg Leu Ser Gly Ser Gly
                   85                  90                  95
   Ser Pro Ala Ser Cys Ser Thr Pro Ala Ser Ser Thr Pro Leu Thr Ile
               100                 105                 110
   Lys Glu Glu Glu Ser Asp Ser Val Ile Gly Asp Met Ser Phe His Asn
           115                 120                 125
   Gln Thr His Thr Thr Asn Glu Glu Glu Glu Ala Glu Glu Asp Asp Asp
       130                 135                 140
   Ile Asp Val Asp Val Asp Asp Thr Ser Ala Gly Gly Arg Leu Pro Pro
   145                 150                 155                 160
   Pro Ala His Gln Gln Gln Ser Thr Ala Lys Pro Ser Leu Ala Phe Ser
                   165                 170                 175
   Ile Ser Asn Ile Leu Ser Asp Arg Phe Gly Asp Val Gln Lys Pro Gly
               180                 185                 190
   Lys Ser Met Glu Asn Gln Ala Ser Ile Phe Arg Pro Phe Glu Ala Ser
           195                 200                 205
   Arg Ser Gln Thr Ala Thr Pro Ser Ala Phe Thr Arg Val Asp Leu Leu
       210                 215                 220
   Glu Phe Ser Arg Gln Gln Gln Ala Ala Ala Ala Ala Ala Thr Ala Ala
   225                 230                 235                 240
   Met Met Leu Glu Arg Ala Asn Phe Leu Asn Cys Phe Asn Pro Ala Ala
   245 250 255
   Tyr Pro Arg Ile His Glu Glu Ile Val Gln Ser Arg Leu Arg Arg Ser
   260 265 270
   Ala Ala Asn Ala Val Ile Pro Pro Pro Met Ser Ser Lys Met Ser Asp
   275 280 285
   Ala Asn Pro Glu Lys Ser Ala Leu Gly Ser Leu Cys Lys Ala Val Ser
   290 295 300
   Gln Ile Gly Gln Pro Ala Ala Pro Thr Met Thr Gln Pro Pro Leu Ser
   305 310 315 320
   Ser Ser Ala Ser Ser Leu Ala Ser Pro Pro Pro Ala Ser Asn Ala Ser
   325 330 335
   Thr Ile Ser Ser Thr Ser Ser Val Ala Thr Ser Ser Ser Ser Ser Ser
   340 345 350
   Ser Gly Cys Ser Ser Ala Ala Ser Ser Leu Asn Ser Ser Pro Ser Ser
   355 360 365
   Arg Leu Gly Ala Ser Gly Ser Gly Val Asn Ala Ser Ser Pro Gln Pro
   370 375 380
   Gln Pro Ile Pro Pro Pro Ser Ala Val Ser Arg Asp Ser Gly Met Glu
   385 390 395 400
   Ser Ser Asp Asp Thr Arg Ser Glu Thr Gly Ser Thr Thr Thr Glu Gly
   405 410 415
   Gly Lys Asn Glu Met Trp Pro Ala Trp Val Tyr Cys Thr Arg Tyr Ser
   420 425 430
   Asp Arg Pro Ser Ser Gly Pro Arg Tyr Arg Arg Pro Lys Gln Pro Lys
   435 440 445
   Asp Lys Thr Asn Asp Glu Lys Arg Pro Arg Thr Ala Phe Ser Ser Glu
   450 455 460
   Gln Leu Ala Arg Leu Lys Arg Glu Phe Asn Glu Asn Arg Tyr Leu Thr
   465 470 475 480
   Glu Arg Arg Arg Gln Gln Leu Ser Ser Glu Leu Gly Leu Asn Glu Ala
   485 490 495
   Gln Ile Lys Ile Trp Phe Gln Asn Lys Arg Ala Lys Ile Lys Lys Ser
   500 505 510
   Thr Gly Ser Lys Asn Pro Leu Ala Leu Gln Leu Met Ala Gln Gly Leu
           515                 520                 525
   Tyr Asn His Thr Thr Val Pro Leu Thr Lys Glu Glu Glu Glu Leu Glu
       530                 535                 540
   Met Arg Met Asn Gly Gln Ile Pro
   545                 550
<210> 9
   <211> 8
   <212> PRT
   <213> Artificial Sequence
<220>
   <223> tag
<400> 9
   Asp Tyr Lys Asp Asp Asp Asp Lys